# EUROPÄISCHE PATENTANMELDUNG

(11) **EP 4 420 627 A1**
(43) Veröffentlichungstag der Anmeldung: **28.08.2024**
(21) Anmeldenummer: 24156482.2
(22) Anmeldetag: 08.02.2024
(51) Int. Cl.: A61B 18/14, A61B 18/04, H01B 1/12

(54) **ELEKTRISCHER LEITER FÜR EIN ELEKTROCHIRURGISCHES INSTRUMENT UND VERFAHREN ZU DESSEN HERSTELLUNG, ELEKTROCHIRURGISCHES INSTRUMENT, ELEKTROCHIRURGISCHE VORRICHTUNG SOWIE VERWENDUNG VON EINEM MATERIAL ZUR HERSTELLUNG EINES ELEKTRISCHEN LEITERS**

(30) Priorität: 22.02.2023 DE 102023104347
(71) Anmelder: Schunk Kohlenstofftechnik GmbH, 35452 Heuchelheim (DE)
(72) Erfinder: GÄRTNER, Ralf, 35633 Lahnau (DE); GÜNTHER, Stefan, 35452 Heuchelheim (DE)
(74) Vertreter: advotec.

(57) **Zusammenfassung**

Die Erfindung betrifft einen elektrischen Leiter für ein elektrochirurgisches Instrument (11), insbesondere zur Koagulation von einem biologischen Gewebe, sowie ein Verfahren und eine Verwendung eines Materials zu dessen Herstellung, wobei der elektrische Leiter aus einem Material auf Basis von einem nachwachsenden Rohstoff hergestellt ist.

## Beschreibung

Die Erfindung betrifft einen elektrischen Leiter für ein elektrochirurgisches Instrument, insbesondere zur Koagulation von einem biologischen Gewebe, und ein Verfahren zu dessen Herstellung. Weiter betrifft die Erfindung ein elektrochirurgisches Instrument, eine elektrochirurgische Vorrichtung und eine Verwendung von einem Material zur Herstellung eines elektrischen Leiters.

Ein für ein elektrochirurgisches Instrument vorgesehener elektrischer Leiter der eingangs bezeichneten Art ist aus dem Stand der Technik hinlänglich bekannt.

Beispielsweise geht aus der EP 3 984 480 A1 ein als eine flexible Sonde ausgebildetes elektrochirurgisches Instrument zur Argon-Plasma-Koagulation von einem biologischen Gewebe hervor, wobei das elektrochirurgische Instrument einen aus einem Metall bzw. rostfreien Stahl ausgebildeten elektrischen Leiter und einen aus Kunststoff ausgebildeten Schlauch umfasst, in welchem der elektrische Leiter angeordnet bzw. aufgenommen ist. Das elektrochirurgische Instrument bzw. der Schlauch ist dabei an eine Gasversorgungseinrichtung anschließbar. Weiter ist das elektrochirurgische Instrument an eine Spannungsversorgungseinrichtung anschließbar, wobei der elektrische Leiter einen an die Spannungsversorgungseinrichtung anschließbaren Zuleitungsabschnitt und ferner einen sich in einer Längserstreckungsrichtung des elektrischen Leiters an den Zuleitungsabschnitt elektrisch leitend anschließenden, im Wesentlichen endseitig an dem elektrischen Leiter angeordneten Elektrodenabschnitt aufweist. In einem Betrieb des elektrochirurgischen Instruments wird der Schlauch mit einem Gas, insbesondere mit einem Edelgas, beispielsweise Argon, und der Elektrodenabschnitt über den Zuleitungsabschnitt mit einer elektrischen Spannung versorgt, wobei der von dem Gas umspülte Elektrodenabschnitt bzw. eine Spitze des Elektrodenabschnitts einen in einem Bereich des Elektrodenabschnitts bzw. der Spitze endseitig aus dem Schlauch austretenden Gasstrom zur Erzeugung eines Plasmastroms ionisiert. Mittels des Plasmastroms wird dann zwischen dem Elektrodenabschnitt bzw. der Spitze und dem biologischen Gewebe ein elektrischer Stromfluss erzeugt, wobei eine mit dem elektrischen Stromfluss verbundene Wärmeentwicklung zu einer Protondenaturierung führt, infolge welcher eine Koagulation des biologischen Gewebes erfolgt.

Nach einer einmaligen Verwendung des elektrochirurgischen Instruments muss das elektrochirurgische Instrument aufgrund von geltenden Hygienebestimmungen thermisch entsorgt bzw. verbrannt werden. Es hat sich als nachteilhaft herausgestellt, dass das elektrochirurgische Instrument bzw. der elektrische Leiter infolge der regelmäßigen Ausbildung des elektrischen Leiters aus dem Metall bzw. rostfreien Stahl nicht rückstandslos verbrennt. Eine Entsorgung gestaltet sich daher aufwendig und wenig nachhaltig.

Der vorliegenden Erfindung liegt daher die Aufgabe zugrunde, einen elektrischen Leiter für ein elektrochirurgisches Instrument, insbesondere zur Koagulation von einem biologischen Gewebe, ein elektrochirurgisches Instrument, eine elektrochirurgische Vorrichtung, ein Verfahren zur Herstellung eines elektrischen Leiters und eine Verwendung von einem Material zur Herstellung eines elektrischen Leiters vorzuschlagen, welcher bzw. welche bzw. welches eine verbesserte Entsorgung des elektrischen Leiters bzw. elektrochirurgischen Instruments ermöglicht.

Diese Aufgabe wird durch einen elektrischen Leiter für ein elektrochirurgisches Instrument, insbesondere zur Koagulation von einem biologischen Gewebe, mit den Merkmalen des Anspruchs 1, ein elektrochirurgisches Instrument mit den Merkmalen des Anspruchs 9, eine elektrochirurgische Vorrichtung mit den Merkmalen des Anspruchs 13, ein Verfahren zur Herstellung eines elektrischen Leiters mit den Merkmalen des Anspruchs 14 und eine Verwendung von einem Material zur Herstellung eines elektrischen Leiters mit den Merkmalen des Anspruchs 15 gelöst.

Der erfindungsgemäße elektrische Leiter für ein elektrochirurgisches Instrument, insbesondere zur Koagulation von einem biologischen Gewebe, ist aus einem Material auf Basis von einem nachwachsenden Rohstoff hergestellt.

Demnach ist erfindungsgemäß vorgesehen, dass der elektrische Leiter aus einem Material auf Basis von einem nachwachsenden Rohstoff bzw. von nachwachsenden Rohstoffen hergestellt bzw. ausgebildet ist, wodurch eine verbesserte, insbesondere rückstandslose und zudem im Wesentlichen klimaneutrale bzw. CO2-neutrale, thermische Entsorgung des elektrischen Leiters ermöglicht wird.

Vorteilhafterweise kann der elektrische Leiter aus dem Material auf Basis von Cellulose hergestellt sein. Die Cellulose kann ganz einfach aus einem Pflanzenmaterial erhalten werden.

Vorteilhafterweise kann das Material ein Fasermaterial mit Fasern sein. Vorteilhafterweise kann das Fasermaterial dann ein Naturfasermaterial mit Naturfasern, insbesondere Cellulosefasern, sein. Alternativ kann das Fasermaterial auch ein Chemiefasermaterial mit Chemiefasern sein, insofern es sich um ein Chemiefasermaterial auf Basis von einem nachwachsenden Rohstoff handelt. Insbesondere können die Chemiefasern dann Regeneratfasern, insbesondere Cellulose-Regeneratfasern, sein. Bei dem Material bzw. Chemiefasermaterial kann es sich dann um Kunstseide handeln. Weiter kann das Fasermaterial ein Faserverbundmaterial sein, wobei die Fasern in eine Matrix, beispielsweise eine Matrix aus Harz, eingebettet sein können.

Weiter kann das Material bzw. Fasermaterial, insbesondere die Cellulose bzw. Cellulosefasern, zur Umwandlung des Materials bzw. Fasermaterials in Kohlenstoff bzw. Kohlenstofffasern karbonisiert sein. Mittels einer Pyrolyse bzw. Temperaturbehandlung kann das Material bzw. Fasermaterial in einer inerten Atmosphäre karbonisiert werden, wodurch das Material bzw. Fasermaterial teilweise oder vollständig in Kohlenstoff umgewandelt werden kann. Folglich kann der elektrische Leiter aus Kohlenstoff bzw. Kohlenstofffasern hergestellt bzw. ausgebildet sein, wobei der Kohlenstoff bzw. die Kohlenstofffasern durch eine Karbonisierung des Materials bzw. Fasermaterials aus dem nachwachsenden Rohstoff erhalten werden kann bzw. können. Bedingt durch eine vergleichsweise hohe elektrische Leitfähigkeit des Kohlenstoffs kann so ein elektrischer Leiter mit einer vergleichsweise hohen elektrischen Leitfähigkeit erhalten werden.

Ferner kann das Material bzw. Fasermaterial mit pyrolytischem Kohlenstoff beschichtet, vorzugsweise infiltriert, sein. Durch die Beschichtung bzw. Infiltration des Fasermaterials mit dem pyrolytischen Kohlenstoff können die Fasern des Fasermaterials, insbesondere die Kohlenstofffasern, untereinander verbunden werden. Eine Matrix kann dann von dem pyrolytischen Kohlenstoff ausgebildet sein. Der pyrolytische Kohlenstoff kann aus einer Gasphase auf den Fasern abgeschieden werden, beispielsweise mittels eines CVD-Verfahrens bzw. eines PVD-Verfahrens bei der Beschichtung oder mittels eines CVI-Verfahrens bzw. eines PVI-Verfahrens bei der Infiltration.

Vorteilhafterweise kann der elektrische Leiter als ein Garn ausgebildet sein. Das Garn kann gezwirnt sein. Vorteilhafterweise kann der elektrische Leiter dann in Gestalt einer Kordel ausgebildet sein. Auch kann der elektrische Leiter in Gestalt eines Filamentgarns ausgebildet sein. Der elektrische Leiter kann in Gestalt des Garns dann ganz einfach abgelängt werden.

Der elektrische Leiter kann vorteilhafterweise linear ausgebildet sein. Beispielsweise kann der Leiter dann eine kreisförmige Querschnittsform aufweisen.

Weiter kann der elektrische Leiter einen Zuleitungsabschnitt und einen Elektrodenabschnitt aufweisen. Der Elektrodenabschnitt kann sich in einer Längserstreckungsrichtung des elektrischen Leiters elektrisch leitend an den Zuleitungsabschnitt anschließen und im Wesentlichen endseitig an dem elektrischen Leiter angeordnet bzw. ausgebildet sein.

Vorteilhafterweise kann der elektrische Leiter einteilig ausgebildet sein. Der Elektrodenabschnitt kann dann einteilig mit dem Zuleitungsabschnitt ausgebildet sein. Die Herstellung des elektrischen Leiters bzw. eines den elektrischen Leiter umfassenden elektrochirurgischen Instruments ist dann besonders einfach, wobei zur Herstellung des elektrochirurgischen Instruments beispielsweise ein Schlauch des elektrochirurgischen Instruments, welcher aus einem Kunststoffmaterial ausgebildet werden kann, auf den elektrischen Leiter extrudiert werden kann. Zudem kann das elektrochirurgische Instrument bzw. der Schlauch mit dem darin angeordneten bzw. aufgenommenen elektrischen Leiter bzw. eine Sonde, als welche das elektrochirurgische Instrument ausgebildet sein kann, dann beliebig und einfach abgelängt werden. Jedoch kann auch vorgesehen sein, den Elektrodenabschnitt gesondert von dem Zuleitungsabschnitt herzustellen und an diesem zu befestigen, so dass der elektrische Leiter dann zumindest zweiteilig ausgebildet sein kann.

Ferner kann der Zuleitungsabschnitt an eine Spannungsversorgungseinrichtung anschließbar sein. Mit einem ersten Ende des Zuleitungsabschnitts kann der Zuleitungsabschnitt dann an der Spannungsversorgungseinrichtung angeschlossen werden, wobei sich der Elektrodenabschnitt in der Längserstreckungsrichtung des elektrischen Leiters an ein zweites Ende des Zuleitungsabschnitts elektrisch leitend anschließen kann, so dass an dem Elektrodenabschnitt über den Zuleitungsabschnitt eine elektrische Spannung anliegen kann.

Das erfindungsgemäße elektrochirurgische Instrument, insbesondere zur Koagulation von einem biologischen Gewebe, umfasst einen erfindungsgemäßen elektrischen Leiter und einen Schlauch, in welchem der elektrische Leiter angeordnet ist.

Vorteilhafterweise kann das elektrochirurgische Instrument nur einen einzigen elektrischen Leiter aufweisen, also monopolar ausgebildet sein. Jedoch kann das elektrochirurgische Instrument grundsätzlich auch mehrere elektrische Leiter aufweisen, also multipolar, insbesondere bipolar, ausgebildet sein.

Das elektrochirurgische Instrument kann vorteilhafterweise als eine Sonde ausgebildet sein.

Der Schlauch kann flexibel oder starr ausgebildet sein. Vorteilhafterweise kann der Schlauch flexibel ausgebildet sein. Der Schlauch kann auch nur abschnittsweise flexibel oder starr ausgebildet sein.

Das elektrochirurgische Instrument kann zur (elektro-)chirurgischen Behandlung eines Patienten durch einen Arbeitskanal eines Endoskops in den Patienten eingeführt werden.

Ferner kann das elektrochirurgische Instrument bzw. der Zuleitungsabschnitt an eine Spannungsversorgungseinrichtung anschließbar sein, welche eine hochfrequente Wechselspannung erzeugen kann.

Weiter kann das elektrochirurgische Instrument bzw. der Schlauch an eine Gasversorgungseinrichtung anschließbar sein, welche ein Gas, insbesondere ein Edelgas, beispielsweise Argon, ausgeben kann.

In einem Betrieb des elektrochirurgischen Instruments kann der Schlauch mit einem Gas, insbesondere mit einem Edelgas, beispielsweise Argon, und der Elektrodenabschnitt über den Zuleitungsabschnitt mit einer elektrischen Spannung versorgt werden, wobei der von dem Gas umspülte Elektrodenabschnitt bzw. eine Spitze des Elektrodenabschnitts einen in einem Bereich des Elektrodenabschnitts der Spitze endseitig aus dem Schlauch austretenden Gasstrom zur Erzeugung eines Plasmastroms ionisieren kann. Mittels des Plasmastroms kann dann zwischen dem Elektrodenabschnitt bzw. der Spitze und einem biologischen Gewebe ein elektrischer Stromfluss erzeugt werden, wobei eine mit dem elektrischen Stromfluss verbundene Wärmeentwicklung zu einer Protondenaturierung führen kann, infolge welcher eine Koagulation des biologischen Gewebes erfolgen kann. Insbesondere kann mittels des chirurgischen Instruments dann eine Argon-Plasma-Koagulation erfolgen.

Ferner kann der Schlauch aus einem Kunststoffmaterial, insbesondere aus einem bio-basierten Kunststoff, ausgebildet sein. Vorteilhafterweise kann der bio-basierte Kunststoff dann ebenfalls auf Basis eines nachwachsenden Rohstoffs hergestellt sein, wodurch eine verbesserte, insbesondere im Wesentlichen klimaneutrale bzw. CO2-neutrale, thermische Entsorgung des gesamten elektrochirurgischen Instruments ermöglicht wird. Jedoch kann der Schlauch auch aus einem nicht bio-basierten Kunststoffmaterial, beispielsweise aus PTFE oder PP, ausgebildet sein.

Die erfindungsgemäße elektrochirurgische Vorrichtung, insbesondere zur Koagulation von einem biologischen Gewebe, umfasst ein erfindungsgemäßes elektrochirurgisches Instrument, eine Spannungsversorgungseinrichtung und eine Gasversorgungseinrichtung. Die Spannungsversorgungseinrichtung und die Gasversorgungseinrichtung können Bestandteil einer Versorgungseinheit sein.

Bei dem erfindungsgemäßen Verfahren zur Herstellung eines elektrischen Leiters für ein elektrochirurgisches Instrument, insbesondere zur Koagulation von einem biologischen Gewebe, wird der elektrische Leiter aus einem Material auf Basis von einem nachwachsenden Rohstoff hergestellt.

Zu den vorteilhaften Wirkungen des erfindungsgemäßen Verfahrens wird auf die Vorteilsbeschreibung des erfindungsgemäßen elektrischen Leiters verwiesen.

Weitere vorteilhafte Ausführungsformen des Verfahrens ergeben sich aus den Merkmalsbeschreibungen der auf den Erzeugnisanspruch 1 rückbezogenen Unteransprüche.

Erfindungsgemäß wird ein Material auf Basis von einem nachwachsenden Rohstoff zur Herstellung eines elektrischen Leiters für ein elektrochirurgisches Instrument, insbesondere zur Koagulation von einem biologischen Gewebe, verwendet.

Zu den vorteilhaften Wirkungen der erfindungsgemäßen Verwendung wird auf die Vorteilsbeschreibung des erfindungsgemäßen elektrischen Leiters verwiesen.

Weitere vorteilhafte Ausführungsformen der Verwendung ergeben sich aus den Merkmalsbeschreibungen der auf den Erzeugnisanspruch 1 rückbezogenen Unteransprüche.

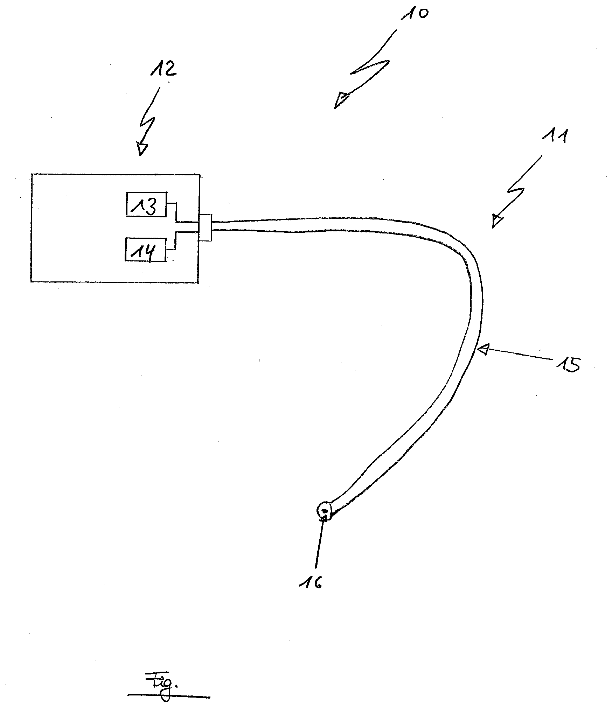

Nachfolgend wird eine bevorzugte Ausführungsform der Erfindung unter Bezugnahme auf die beigefügte Zeichnung näher erläutert.

Die **Fig.** zeigt eine schematische Darstellung einer elektrochirurgischen Vorrichtung 10 zur Koagulation von einem biologischen Gewebe, umfassend ein elektrochirurgisches Instrument 11 und eine Versorgungseinheit 12, an welche das elektrochirurgische Instrument 11 angeschlossen ist, mit einer Spannungsversorgungseinrichtung 13 und einer Gasversorgungseinrichtung 14. Das als eine Sonde ausgebildete elektrochirurgische Instrument 11 umfasst einen Schlauch 15 und einen elektrischen Leiter, welcher in dem Schlauch 15 angeordnet bzw. aufgenommen ist. Dabei ist von dem elektrischen Leiter lediglich eine Spitze 16 eines Elektrodenabschnitts des elektrischen Leiters sichtbar. Der elektrische Leiter ist aus einem Material auf Basis von einem nachwachsenden Rohstoff hergestellt, wodurch eine verbesserte thermische Entsorgung der aufgrund von geltenden Hygienebestimmungen zu einer einmaligen Verwendung vorgesehenen Sonde ermöglicht wird.

In einer vorteilhaften Ausführungsform der Erfindung kann der elektrische Leiter aus einem karbonisierten und mit pyrolytischem Kohlenstoff infiltrierten Naturfasermaterial, vorzugsweise Cellulosefasern, ausgebildet sein. Vorzugsweise kann der elektrische Leiter dabei in Gestalt einer Kordel ausgebildet sein.

## Patentansprüche

1. Elektrischer Leiter für ein elektrochirurgisches Instrument (11), insbesondere zur Koagulation von einem biologischen Gewebe,
**dadurch gekennzeichnet,**
**dass** der elektrische Leiter aus einem Material auf Basis von einem nachwachsenden Rohstoff hergestellt ist.

2. Elektrischer Leiter nach einem der vorangehenden Ansprüche,
**dadurch gekennzeichnet,**
**dass** der elektrische Leiter aus dem Material auf Basis von Cellulose hergestellt ist.

3. Elektrischer Leiter nach Anspruch 1 oder 2,
**dadurch gekennzeichnet,**
**dass** das Material ein Fasermaterial, insbesondere ein Naturfasermaterial mit Naturfasern, ist.

4. Elektrischer Leiter nach einem der vorangehenden Ansprüche,
**dadurch gekennzeichnet,**
**dass** das Material karbonisiert ist.

5. Elektrischer Leiter nach einem der vorangehenden Ansprüche,
**dadurch gekennzeichnet,**
**dass** das Material mit pyrolytischem Kohlenstoff beschichtet, vorzugsweise, infiltriert ist.

6. Elektrischer Leiter nach einem der vorangehenden Ansprüche,
**dadurch gekennzeichnet,**
**dass** der elektrische Leiter als ein Garn ausgebildet ist.

7. Elektrischer Leiter nach einem der vorangehenden Ansprüche,
**dadurch gekennzeichnet,**
**dass** der elektrische Leiter einen Zuleitungsabschnitt und einen Elektrodenabschnitt aufweist.

8. Elektrischer Leiter nach einem der vorangehenden Ansprüche,
**dadurch gekennzeichnet,**
**dass** der elektrische Leiter einteilig ausgebildet ist.

9. Elektrochirurgisches Instrument (11), insbesondere zur Koagulation von einem biologischen Gewebe, umfassend einen elektrischen Leiter nach einem der vorangehenden Ansprüche und einen Schlauch (15), in welchem der elektrische Leiter angeordnet ist.

10. Elektrochirurgisches Instrument nach Anspruch 9,
**dadurch gekennzeichnet,**
**dass** das elektrochirurgische Instrument (11) an eine Spannungsversorgungseinrichtung (13) anschließbar ist.

11. Elektrochirurgisches Instrument nach Anspruch 9 oder 10,
**dadurch gekennzeichnet,**
**dass** das elektrochirurgische Instrument (11) an eine Gasversorgungseinrichtung (14) anschließbar ist.

12. Elektrochirurgisches Instrument nach einem der Ansprüche 9 bis 11, **dadurch gekennzeichnet,**
**dass** der Schlauch (15) aus einem Kunststoffmaterial, insbesondere aus einem bio-basierten Kunststoff, ausgebildet ist.

13. Elektrochirurgische Vorrichtung (10), insbesondere zur Koagulation von einem biologischen Gewebe, umfassend ein elektrochirurgisches Instrument (11) nach einem der Ansprüche 9 bis 12, eine Spannungsversorgungseinrichtung (13) und eine Gasversorgungseinrichtung (14).

14. Verfahren zur Herstellung eines elektrischen Leiters für ein elektrochirurgisches Instrument (11), insbesondere zur Koagulation von einem biologischen Gewebe,
**dadurch gekennzeichnet,**
**dass** der elektrische Leiter aus einem Material auf Basis von einem nachwachsenden Rohstoff hergestellt wird.

15. Verwendung von einem Material auf Basis von einem nachwachsenden Rohstoff zur Herstellung eines elektrischen Leiters für ein elektrochirurgisches Instrument (11), insbesondere zur Koagulation von einem biologischen Gewebe.
